# EUROPEAN PATENT APPLICATION

(11) **EP 4 484 958 A1**
(43) Date of publication of application: **01.01.2025**
(21) Application number: 23182541.5
(22) Date of filing: 29.06.2023
(51) Int. Cl.: G01N 33/68

(54) **TARGETED PROTEOMICS FOR MONITORING AUTOPHAGY**

(71) Applicant: University Of Fribourg, 1700 Fribourg (CH)
(72) Inventor: The designation of the inventor has not yet been filed
(74) Representative: Stolmár & Partner Patentanwälte PartG mbB

(57) **Abstract**

The invention relates to a peptide mix comprising peptides for use in monitoring autophagy, the peptides being derived from proteins involved in autophagy.

## Description

### Field of the invention

The invention relates to peptide mixes for use in mass spectrometry.

### Technical background

Eukaryotic cells maintain homeostasis and remove damaged or superfluous cellular components through a conserved degradation pathway called macroautophagy (hereafter: autophagy). This highly conserved catabolic pathway occurs at basal levels but is enhanced by a variety of stress signals, such as nutrient starvation or organelle damage. The process starts with the de-novo formation of a double-membrane organelle called autophagosome from membranes being recruited from various sources, the endoplasmic reticulum (ER) likely being principle donor. Autophagosome biogenesis and turnover is a directional process and can be divided into five principle stages.

In phase 1 "autophagosome initiation" kinase complexes are activated that start the process. In phase 2 "membrane nucleation" initial membrane sources are recruited to the phagophore assembly site, the principal site of autophagosome biogenesis.

In phase 3 "membrane expansion" and phase 4 "pore closure", the growing cup-shaped membrane engulfs parts of the cytoplasm and closes up to form a double membrane vesicle. The autophagosome can fuse with other vesicles from endocytic pathways forming an amphisome and finishes in phase 5 by "fusing with the lysosome/vacuole". Lysosomal fusion exposes autophagosomal cargo including the inner membrane to acidic hydrolases and enables the degradation of its content. The generated building blocks are recycled and transported back to the cytosol by lysosomal permeases to generate energy or fuel anabolic pathways.

While basal autophagy is often considered a "bulk process", i.e., a non-selective process recycling cellular components in a random manner, autophagy can also be selective. Well studied cases of selective autophagy include the targeted removal of damaged organelles such as the mitochondrion or ER (termed mitophagy and reticulophagy/ER-phagy, respectively) as well as of protein aggregates (aggrephagy). This particular selectivity is enabled by a set of proteins called selective autophagy receptors (SARs), bridging the autophagy cargo to proteins of the ATG8 family, which are lipidated proteins coating the autophagosomal membrane and functioning as docking sites.

Numerous methods and protocols have been described for the study of protein turnover by autophagy, several of them relying on immunodetection or fluorescent microscopy of single proteins, which are often ectopically expressed as tagged variants facilitating downstream analyses. While these methods are well established, they often require a significant protein amount or protein tagging, making them unsuitable for some sample types, e.g. primary cells being difficult to transfect, and difficult to adapt for large-scale screening approaches. Additionally, some of these methods infer the activity of the whole pathway based on the quantification of single markers. Whereas this might be relevant for the analysis of basal, i.e. non-selective, autophagy flux, such approaches likely fail to capture the complexity of stress-induced autophagy in which different cargoes might be degraded at different rates and through different pathways.

To address the complexity of autophagy regulation and activity in an unbiased manner, approaches monitoring the activity of multiple genes by RNA-seq have been developed that are amendable for higher sample throughput and might also be used in prognostic/diagnostic settings. However, to infer autophagy activity based on changes in gene transcription biological samples have to be treated for several hours, commonly 2-8 hours, which is in contrast to the rapid cellular response to stress conditions. Autophagy signaling is activated within minutes, initial autophagosome biogenesis does not require de novo protein synthesis, and in mammalian cells autophagosomes and autophagy-dependent protein degradation can be detected as early as 30 min post stimulus.

### Objective problem to be solved

To address this gap in analytical approaches, an assay is required that is able to detect changes in protein abundance, which is amenable to high throughput and which monitors abundance changes of multiple endogenous proteins reflecting the complexity and multitude of autophagy subtypes.

### Summary of the invention

In one aspect, the invention relates to a peptide mix comprising peptides for use in monitoring autophagy, the peptides being derived from proteins involved in autophagy.

In a second aspect, the invention relates to use of the peptide mix according the invention as a peptide standard for mass spectrometry.

In a third aspect, the invention relates to a method for monitoring autophagy, the method comprising using the peptide mix according to any of claims 1 to 12 as a peptide standard for mass spectrometry.

### Brief description of the figures

Fig. 1A-B shows examples of calibration curves for two peptides belonging to the GABARAPL2 protein. Left-side corresponds to intensity-concentration responses with low concentrations in the insets. Right side is a schematic representation of the location of each peptide in the protein.
**Fig. 2** shows the setup experiment. **A.** Average CV per technical replicate, **B.** Heatmap representing z-scored normalized peak intensities for each of the peptide that could be accurately measured. C. individual peptide values for peptides of the TAX1 BP1 protein.
**Fig. 3** shows protein regulation by autophagy upon different starvation stimuli. **A.** Heatmap summarizing the behavior of regulated proteins reaching a p-value < 0.01 in an ANOVA test across all conditions. **B.** Example proteins for the main three identified clusters in the experiment. Basal conditions (DMEM) were set as 1 for reference.

### Detailed description of the invention

The objective problem is solved by a peptide mix comprising isotopically labelled heavy peptides for use in monitoring autophagy, the peptides being derived from proteins involved in autophagy.

As used herein, the term "peptide" refers to an amino acid chain having a length of between 5 and 40 amino acids. In a preferred embodiment, the peptides have length between 7 and 25 amino acids.

The peptides used in the peptide mix according to the invention are isotopically labelled heavy peptides. The skilled person knows how peptides can be isotopically labelled. In some embodiments, the peptides are labelled with ¹³C, ¹⁵N or ²H. In a preferred embodiment, the peptides are labelled with ¹³C and/or ¹⁵N. In principle, any amino acid within the peptides may be labelled. In preferred embodiments, it is the arginine and lysine residues that are labelled.

According to the invention, the peptides contained in the peptide mix represent various key proteins involved in different stages of autophagy.

In a preferred embodiment, the peptides contained in the peptide mix according to the invention are derived from proteins of one of seven classes, namely proteins involved in autophagy regulation, proteins involved in autophagosome initiation, proteins involved in membrane nucleation, proteins involved in membrane expansion, proteins involved in pore closure, proteins involved in fusion of the autophagosome with lysosomes and selective autophagy receptors (SAR) proteins.

As used herein, the term "peptide derived from a protein" refers to a peptide sequence that corresponds to a peptide generated from a particular protein by proteolysis. Proteases used for generating such peptides are LysC, AspN, GluC, chymotrypsin, proalanase, ArgC, elastase, pepsin and trypsin. In a preferred embodiment, the peptides used in the peptide mix according to the invention are tryptic peptides, i.e., a peptide sequence that can be generated by treating the protein of interest with the endoprotease trypsin, which cuts amino acid chains after lysine and arginine residues if they are not followed by proline.

The peptides used in the peptide mix according to the invention may be non-modified or posttranslationally modified peptides. In one embodiment, the peptides are modified in the same way as proteins are modified posttranslationally, e.g., by phosphorylation, acetylation, methylation or ubiquitination. Autophagy is regulated on a posttranslational level and protein activity is modulated by phosphorylation, acetylation, methylation, and ubiquitination. Therefore, by including modified peptides in the peptide mix, the peptide mix according to the invention will be better suited to detect different forms of the proteins involved in autophagy.

The peptide mix according to the invention can be used in mass spectrometry (MS)-based analyses which support absolute protein quantification via accurate quantification of a predefined number of given peptides potentially encompassing numerous proteins. Quantification can be performed in targeted and non-targeted MS approaches. Non-targeted approaches such as Data Dependent Acquisition (DDA) and Data Independent Acquisition (DIA) monitor potentially all peptides being present in a given sample. Mass spectrometers operated in targeted mode focus on a pre-established set of mass-to-charge ratios corresponding to peptides-of-interest. Targeted approaches can be inclusive or exclusive, i.e. focusing on peptides-of-interest AND other peptides being present, or focusing ONLY on peptides-of-interest, respectively. By ignoring other peptides eluting from the coupled liquid chromatography (LC) column (exclusive), targeted proteomics greatly enhances the sensitivity for its targets. Combining this approach with the peptide mix according to the invention as spiked-in peptides further enables confident identification of the targets and robust normalization across samples, thus making the method suitable for absolute quantification.

More specifically, the peptide mix according to the invention can be used in a targeted proteomics approach, parallel reaction monitoring (PRM) or multiple reaction monitoring (MRM) or single reaction monitoring (SRM), allowing to accurately quantify tryptic peptides derived from human autophagy-relevant proteins in single MS measurements. In combination with a simple in-solution digestion protocol that can be performed on multiple, low protein concentration samples (atto- to millimolar concentrations) in a (semi)automatic manner the assay supports screening approaches.

In the peptide mix, the peptides are dissolved in any regular mass spectrometry buffer known in the art, e.g., in 50 mM ammonium bicarbonate buffer or in 0.1% formic acid in water.

According to the invention, proteins involved in autophagy regulation are MTOR/FRAP, RPTOR, RICTOR, MLST8, AMPK (PRKAA1, PRKAB1, PRKAG1), ribosomal S6 kinase (RPS6KA1, RPS6KB1), TFE3 and TFEB. Therefore, in one embodiment, the peptide mix comprises or consists of peptides derived from ribosomal S6 kinase (RPS6KB1) and/or TFEB.

According to the invention, proteins involved in autophagosome initiation comprise ATG9A, AMBRA1, WBP2, ULK1, ULK2, RB1CC1/FIP200, ATG13, ATG101, PIK3C3/VPS34, PIK3R4/VPS15, BECN1/Beclin-1, and ATG14. Therefore, in one embodiment, the peptide mix comprises or consists of peptides derived from ATG9A, AMBRA1, WBP2, ULK1, ULK2, RB1CC1/FIP200 and/or ATG13.

According to the invention, proteins involved in membrane expansion comprise MAP1LC3A, MAP1LC3B, MAP1LC3C, GABARAP, GABARAPL1, GABARAPL2, ATG2A, WIPI1, WIPI2, WIPI3, WIPI4, ATG3, ATG7, ATG12, ATG5 and ATG4A. Therefore, in one embodiment, the peptide mix comprises or consists of peptides derived from ATG2A, WIPI2, ATG3, ATG7, ATG12, ATG5 and/or ATG4A.

According to the invention, proteins involved in pore closure and fusion with lysosomes comprise UVRAG, GABARAPL1 and GABARAPL2. Therefore, in one embodiment, the peptide mix comprises or consists of peptides derived from UVRAG, GABARAPL1 and/or GABARAPL2.

According to the invention, selective autophagy receptors (SAR) proteins comprise SQSTM1/p62, NBR1, OPTN/Optineurin, CALCOCO1, CDK5RAP3/C53, CALCOCO2/NDP52, TAX1BP1, WDFY3/Alfy, CCPG1, FAM134B, RTN3, NIX/BNIP3L, TOLLIP, TNIP1, BNIP3, PHB2, NCOA4, NUFIP1, STBD1, BNIP1, PHBP2, TRIM16, FUNDC1, FKBP8, NLRX1, ATL3, TEX264, SEC62, and PNPLA2. Therefore, in one embodiment, the peptide mix comprises or consists of peptides derived from SQSTM1, NBR1, Optineurin, CALCOCO2, TAX1BP1, Alfy, CCPG1, FAM134B, RTN3, NIX/BNIP3L, TOLLIP, TNIP1, FKBP8, BNIP3, PHB2, NCOA4, NUFIP1, STBD1, BNIP1, PHBP2 and/or PNPLA2.

According to the invention, the peptides are derived from source proteins by *in silico* proteolytic digestion using a suitable endoprotease such as trypsin. Not all peptides from a given source protein are suited for targeted MS as some sequences might not fit the requirements of LC (highly hydrophilic or hydrophobic peptides), chemical stability (e.g. post-lysis methionine oxidation), or distribution of cleavage sites (lysine or arginine positions in the sequence prevent digestion into suitable peptides yielding either too large or too small peptides). Selected peptides need also be screened for their ability to be detected in MS. In particular, the peptides have to be able to be ionized and be stable after ionization. Furthermore, it must be possible to retain the peptide on columns commonly used with MS, e.g., LC C18 column coupled to the mass spectrometer. Lastly, it is important that MS signal generated by the peptides has a broad linear concentration range.

The peptides to be used in the peptide mix are then synthesized by methods known in the art, e.g., solid-phase synthesis.

In a preferred embodiment, the peptide mix comprises at least 7 peptides selected from the group consisting of peptides having SEQ ID NO: 1 to 97.

**Table 1: Validated peptides and their analytical figures of merit.**

| **Gene name** | **Uniprot accession number** | **Peptide Sequence** | **LOD** | **LOQ** | **Fit method** | **SEQ ID NO:** |
|---|---|---|---|---|---|---|
| AMBRA1 | Q9C0C7 | TVGVAFNQETGHWER | 0.00 | 1.26 | Linear | 1 |
| | | WLPEPGLGLAYGTNK | 0.07 | 1.36 | Linear | 2 |
| ATG12 | 094817 | TIQGLIDFIK | 1.71 | 3.15 | Nonlinear | 3 |
| ATG13 | 075143 | ASPHDVLETIFVR | 0.00 | 2.21 | Linear | 4 |
| | | EGGVPLAPNQPVHGTQADQER | 0.07 | 2.37 | Linear | 5 |
| | | SSSSPTGSDWFNLAIK | 0.04 | 1.24 | Linear | 6 |
| ATG2A | Q2TAZ0 | DLADALLDTER | 0.05 | 1.09 | Linear | 7 |
| | | DLLWLPIEQYR | 0.16 | 1.49 | Linear | 8 |
| | | FTSEVPIWLDYHGK | 0.07 | 2.22 | Linear | 9 |
| ATG3 | Q9NT62 | ALEVAEYLTPVLK | 0.17 | 2.02 | Linear | 10 |
| | | FVQAVIPTIEYDYTR | 5.66 | 6.43 | Nonlinear | 11 |
| | | TDAGGEDAILQTR | 0.03 | 1.73 | Linear | 12 |
| ATG4A | Q8WYN0 | LGINQINPVYVDAFK | 2.57 | 4.08 | Nonlinear | 13 |
| | | LLSDISAR | 0.06 | 1.39 | Linear | 14 |
| | | SIGEWFGPNTVAQVLK | 11.12 | 31.64 | Nonlinear | 15 |
| ATG5 | Q9H1Y0 | EAEPYYLLLPR | 0.08 | 1.40 | Linear | 16 |
| | | IPTCFTLYQDEITER | 0.05 | 2.60 | Linear | 17 |
| | | VSYLTLVTDK | 0.03 | 0.82 | Linear | 18 |
| ATG7 | 095352 | DVAHSIIFEVK | 0.06 | 3.53 | Nonlinear | 19 |
| | | GYYYNGDSAGLPAR | 0.04 | 1.79 | Linear | 20 |
| ATG9A | Q7Z3C6 | ALEIIDFFR | 0.42 | 2.96 | Linear | 21 |
| | | LEASYSDSPPGEEDLLVHVAEGSK | 0.00 | 1.64 | Linear | 22 |
| | | SFSPLQPGQAPTGR | 0.03 | 1.65 | Linear | 23 |
| BNIP1 | Q12981 | AELLQGGDLLR | 0.06 | 1.08 | Linear | 24 |
| | | TILDANEEFK | 1.81 | 2.99 | Linear | 25 |
| BNIP3 | Q12983 | ASETDTHSIGEK | 0.03 | 1.10 | Linear | 26 |
| | | ILLDAQHESGR | 0.15 | 1.56 | Linear | 27 |
| BNIP3L | 060238 | DHSSQSEEEVVEGEK | 0.02 | 1.73 | Linear | 28 |
| | | ILLDAQHESGQSSSR | 0.02 | 1.86 | Linear | 29 |
| CALCOCO2 | Q13137 | DYWETELLQLK | 0.02 | 1.33 | Linear | 30 |
| | | ENDHLFLSLTEQR | 0.09 | 1.74 | Linear | 31 |
| CALCOCO2-4 | Q13137 | LEQELLK | 1.66 | 2.53 | Linear | 32 |
| CCPG1 | Q9ULG6 | FFLNGVFIHDQK | 0.07 | 3.38 | Nonlinear | 33 |
| | | GELQQLSGSQLHGK | 0.07 | 1.43 | Linear | 34 |
| | | IPEDSIYIGTASDDSDIVTLEPPK | 21.61 | 51.09 | Nonlinear | 35 |
| FAM134B | Q9H6L5 | LDFGIGEYINQK | 0.11 | 1.90 | Linear | 36 |
| | | SLSESWEVINSKPDERPR | 0.07 | 2.81 | Linear | 37 |
| FKBP8 | Q14318 | ADFVLAANSYDLAIK | 2.98 | 4.18 | Nonlinear | 38 |
| | | VLAQQGEYSEAIPILR | 0.07 | 1.34 | Linear | 39 |
| GABARAPL1 | Q9H0R8 | EDHPFEYR | 0.06 | 1.80 | Linear | 40 |
| GABARAPL2 | P60520 | IQLPSEK | 0.07 | 1.79 | Linear | 41 |
| | | VSGSQIVDIDK | 0.04 | 1.58 | Linear | 42 |
| NBR1 | Q14596 | GALSVAASAYK | 0.01 | 1.63 | Linear | 43 |
| | | IHLWNSIHGLQSPK | 0.29 | 4.38 | Linear | 44 |
| | | VSFDLNTIQIK | 0.13 | 3.55 | Linear | 45 |
| NCOA4 | Q13772 | DLELAIGGVLR | 0.61 | 4.27 | Nonlinear | 46 |
| NUFIP1 | Q9UHK0 | DFFGLDTNSAK | 0.05 | 0.88 | Linear | 47 |
| | | DYHNYQTLFEPR | 0.04 | 1.48 | Linear | 48 |
| OPTN | Q96CV9 | ADLLGIVSELQLK | 4.63 | 31.28 | Nonlinear | 49 |
| | | LNSSGSSEDSFVEIR | 0.16 | 1.69 | Linear | 50 |
| | | TSDSDQQAYLVQR | 0.03 | 2.80 | Linear | 51 |
| PHB2 | Q99623 | IGGVQQDTILAEGLHFR | 0.21 | 3.10 | Linear | 52 |
| | | IVQAEGEAEAAK | 0.00 | 1.15 | Linear | 53 |
| | | LLLGAGAVAYGVR | 0.22 | 1.77 | Linear | 54 |
| PNPLA2 | Q96AD5 | VSDGENVIISHFNSK | 0.15 | 2.17 | Linear | 55 |
| | | YVDGGISDNLPLYELK | 0.06 | 1.21 | Linear | 56 |
| RB1CC1 | Q8TDY2 | ELAQGFLANQK | 0.02 | 1.41 | Linear | 57 |
| | | SLLEQETENLR | 0.04 | 1.22 | Linear | 58 |
| RPS6KB1 | P23443 | FSPGDFWGR | 0.04 | 1.05 | Linear | 59 |
| | | HINWEELLAR | 0.53 | 3.47 | Linear | 60 |
| | | LGAGPGDAGEVQAHPFFR | 0.00 | 1.73 | Linear | 61 |
| RTN3 | 095197 | TTPPVEVLHENESGGSEIK | 0.17 | 1.93 | Linear | 62 |
| RTN3 | 095197 | VEGIYTYSLSPSK | 0.01 | 1.28 | Linear | 63 |
| SQSTM1 | Q13501 | EAALYPHLPPEADPR | 0.00 | 0.00 | Linear | 64 |
| | | LAFPSPFGHLSEGFSHSR | 4.16 | 4.75 | Nonlinear | 65 |
| | | LTPVSPESSSTEEK | 0.04 | 1.74 | Linear | 66 |
| | | NYDIGAALDTIQYSK | 0.09 | 2.21 | Linear | 67 |
| | | VAALFPALR | 0.07 | 1.34 | Linear | 68 |
| STBD1 | 095210 | EHVPSGQFPDTEAPATSETSNSR | 0.06 | 1.38 | Linear | 69 |
| | | FVLVENGGVTR | 0.09 | 1.92 | Linear | 70 |
| | | HSSWGDVGVGGSLK | 0.03 | 2.59 | Linear | 71 |
| TAX1BP1 | Q86VP1 | AHQLEEDIVSVTHK | 0.15 | 3.30 | Linear | 72 |
| | | LSDQSANNNNVFTK | 0.04 | 1.63 | Linear | 73 |
| TFEB | P19484 | ANDLDVR | 0.29 | 2.22 | Linear | 74 |
| | | EYLSETYGNK | 0.07 | 1.95 | Linear | 75 |
| | | FAAHISPAQGSPK | 0.07 | 2.42 | Linear | 76 |
| | | FAAHISPAQGSPKPPPAASPGVR | 0.28 | 4.30 | Linear | 77 |
| TNIP1 | Q15025 | IYDPGGSVPSGEASAAFER | 0.03 | 370.64 | Linear | 78 |
| | | LQAQVTLSNAQLK | 0.04 | 1.41 | Linear | 79 |
| TOLLIP | Q9H0E2 | GPVYIGELPQDFLR | 0.23 | 3.37 | Linear | 80 |
| | | IAWTHITIPESLR | 0.06 | 2.71 | Linear | 81 |
| | | LGYAVYETPTAHNGAK | 10.82 | 21.40 | Nonlinear | 82 |
| ULK1 | 075385 | APFQASSPQDLR | 0.05 | 1.25 | Linear | 83 |
| | | LPDFLQR | 0.05 | 1.21 | Linear | 84 |
| | | TLSEDTIR | 0.03 | 2.21 | Linear | 85 |
| | | TLVPTIPR | 0.07 | 2.08 | Linear | 86 |
| | | TPSSQNLLALLAR | 0.12 | 194.82 | Linear | 87 |
| | | VAELLSSGLQSAIDQIR | 2.91 | 4.39 | Nonlinear | 88 |
| ULK2 | Q8IYT8 | TLSEDTIR | 0.03 | 2.21 | Linear | 89 |
| WBP2 | Q969T9 | AEAGGGWEGSASYK | 1.62 | 53.17 | Linear | 90 |
| | | QPVFGANYIK | 4.64 | 7.05 | Linear | 91 |
| WDFY3 | Q8IZQ1 | DGFIFVNYSEGQTR | 30.88 | 1106.15 | Nonlinear | 92 |
| | | SEGWPSPVSLVPEEK | 0.05 | 1.02 | Linear | 93 |
| WIPI2 | Q9Y4P8 | EKPPEEPTTWTGYFGK | 0.06 | 0.93 | Linear | 94 |
| | | FFSLSSVDK | 0.07 | 1.10 | Linear | 95 |
| | | GTYVPSSPTR | 0.06 | 2.37 | Linear | 96 |
| | | VFSIPEGQK | 0.06 | 1.81 | Linear | 97 |

In another preferred embodiment, the peptide mix comprises at least one peptide derived from a protein involved in autophagy regulation, at least one peptide derived from a protein involved in autophagosome initiation, at least one peptide derived from a protein involved in membrane nucleation, at least one peptide derived from a protein involved in membrane expansion, at least one peptide derived from a protein involved in pore closure, at least one peptide derived from a protein involved in fusion with lysosomes and at least one peptide derived from a selective autophagy receptors (SAR) protein. Thus, the peptide mix comprises at least 7 peptides, preferably selected from the group consisting of peptides having SEQ ID NO: 1 to 97.

In another embodiment, the peptide mix comprises at least two peptides derived from a protein involved in autophagy regulation, at least two peptides derived from a protein involved in autophagosome initiation, at least two peptides derived from a protein involved in membrane nucleation, at least two peptides derived from a protein involved in membrane expansion, at least two peptides derived from a protein involved in pore closure, at least two peptides derived from a protein involved in fusion with lysosomes and at least two peptides derived from a selective autophagy receptors (SAR) protein. In one embodiment, the two peptides in each class are derived from the same protein. In another embodiment, the two peptides are derived from different proteins of that class. The peptides are preferably selected from the group consisting of peptides having SEQ ID NO: 1 to 97.

In one embodiment, the peptide mix consists of one peptide derived from a protein involved in autophagy regulation, one peptide derived from a protein involved in autophagosome initiation, one peptide derived from a protein involved in membrane nucleation, one peptide derived from a protein involved in membrane expansion, one peptide derived from a protein involved in pore closure, one peptide derived from a protein involved in fusion with lysosomes and one peptide derived from a selective autophagy receptors (SAR) protein. The peptides are preferably selected from the group consisting of peptides having SEQ ID NO: 1 to 97.

In another preferred embodiment, the peptide mix comprises or consists of at least one peptide from one, several, or each SAR protein, i.e., SQSTM1, NBR1, Optineurin, CALCOCO2, TAX1BP1, Alfy, CCPG1, FAM134B, RTN3, NIX/BNIP3L, TOLLIP, TNIP1, FKBP8, BNIP3, PHB2, NCOA4, NUFIP1, STBD1, BNIP1, PHBP2, PNPLA2, GABARAPL1 and GABARAPL2. The peptides are preferably selected from the group consisting of peptides having SEQ ID NO: 1 to 97.

In one aspect of the invention, the peptide mix according to the invention is used as a peptide standard for mass spectrometry (MS). Preferably, the MS is liquid chromatography tandem-mass spectrometry (LC-MS/MS). When the peptide mix according to the invention is used as MS standard, it is spiked into, i.e., added at a suitable amount (e.g., 0.01-250 fmol) to a sample of interest, e.g., a tryptic digest of a lysed tissue or cell sample. The peptide mix is used to determine the LC retention times and MS signals of respective endogenous peptides. The extracted ion currents of the peptides of the peptide mix are used to generate mass chromatograms for quantification of peptides of the peptide mix and the endogenous peptides. For quantification either the *area under the curve* or the *intensity maximum* of respective mass chromatograms, series of mass chromatograms, or mass spectra are used.

In another aspect, the invention relates to methods for monitoring autophagy, the method comprising using the peptide mix according to the invention as a peptide standard for mass spectrometry. In one embodiment, the method comprises a step of quantifying proteins involved in autophagy by mass spectrometry.

The proteins that are quantified in the methods according the invention are preferably proteins involved in autophagy regulation, in autophagosome initiation, in membrane nucleation, in membrane expansion, in pore closure, in fusion of the autophagosome with lysosomes and/or SAR proteins.

In a further embodiment, the methods according to the invention comprise a step of extracting the proteins from a biological sample via lysis, a step of digesting the tissue or cell lysate with trypsin or another suitable endopeptidase/endoprotease, a step of purifying the obtained peptides, a step of spiking the obtained peptide sample with the peptide mix (e.g., 0.01-250 fmol) according to the invention, and/or a step of introducing the spiked peptide sample into a mass spectrometer.

### Examples

### Example 1

### Cell culture

A549 lung carcinoma cells were grown in Dulbecco's Modified Eagle Medium (DMEM, PAN biotech) supplemented with 10% Fetal Bovine Serum (FBS, BioWest) and Penicilin Streptamycin (PAN Biotech). 2.2×10⁶ cells were seeded into 10 cm dishes to reach about 80% confluency on the next day. Treatments were performed by washing the plates twice with PBS before changing to the treatment medium for 2 h. Treatment media were Hank's Balanced Salt Solution (HBSS, Thermo Fisher) for amino-acid starvation and DMEM glucose-free (PAN biotech) supplemented with 10% dialyzed FBS (BioWest), penicillin/streptomycin (PAN Biotech) and stable glutamine (GlutaMAX, Thermo Fisher). Concanamycin A (Sigma-Aldrich) was used at a final concentration of 10 µM.

Cells were washed twice with ice-cold PBS before harvesting by scrapping on ice. Cell pellets were snap frozen in liquid nitrogen.

### MS samples preparation

Pellet were lysed in 5% sodium deoxycholate buffer in 50 mM ammonium bicarbonate pH 8.5. Samples were sonicated (Bioruptor, diagenode) at 8°C for 10 min alternating 30 s pulses on medium intensity and 30 s breaks.

Protein concentration was measured and adjusted using BCA assay and samples were spiked with 10 or 100 fmol of heavy-labeled peptides. Samples were subsequently diluted to reduce sodium deoxycholate below 1%. Samples were reduced by adding 1 mM DTT and incubated at 37°C for 30 min, then alkylated with IAA for 15 min in the dark at room temperature for 15 min.

Samples were digested with trypsin to a 1:100 trypsin (Promega) to protein ratio for 15 h at 37°C with constant agitation. Trypsin was inhibited and sodium deoxycholate was precipitated adding 50% TFA to a final concentration of 2%. Samples were desalted using AssayMAP C18 cartridges (Agilent) on a Bravo liquid handling platform (Agilent), and eluted in 50 µl 80% acetonitrile and 0.1% formic acid. Solvents were lyophilized to remove organic solvents. Peptides were resuspended in 0.1% formic acid to a 1 µg/µl.

### Synthetic peptides

Heavy-labelled synthetic standards for all peptides were acquired from SpikeTides (JPT) or custom synthesis (GenScript) with the following chemical modifications: Carbamoyl methylated cysteine, Carboxy-terminal ¹³C₆-¹⁵N₂ Lysines, ¹³C₆-¹⁵N₄ Arginines. Peptides were not purified but isotope label purity was high. Crude peptides were used and the purity of peptides was not taken into account for calculating concentrations.

### LC-MS

LC-MS/MS measurements were performed on an EASY-nLC 1000 nano-flow UHPLC system (Thermo Fisher Scientific) coupled to a Q Exactive HF-X hybrid quadrupole-Orbitrap mass spectrometer (Thermo Fisher Scientific). 5 µl of solubilized peptides in solvent A (0.1% formic acid in water) were separated on a fused silica HPLC column (75 µm internal diameter column Fused-silica PicoTip^{®} emitter: SilicaTip^{™}, New Objective) self-packed with Waters Acquity CSH C18, 1.7 µm (Waters) to a length of 20 cm) using a linear gradient of solvent A and solvent B (0.1% formic acid in 80% acetonitrile in water) from 4% solvent B to 30% over 85 min, followed by an increase to 100% buffer B over 8 min and 7 min at 100% buffer B at a 250 nl/min flow rate. The spray voltage was set to 2.3 kV with a capillary temperature of 250°C.

Mass spectrometer was operated in PRM mode at 60'000 resolution with an AGC target set as 1e6 and a maximum injection time of 118 ms. Isolation window was set at 1.5 m/z, normalized collision energy was set at 27. Data was acquired in centroid mode. Targeted precursors were monitored during 6 min windows in the calibration curve experiments and 3.5 min windows in the later experiments.

Spectral libraries were acquired using a mixture of the synthetic peptides of reference in Data Dependent acquisition with the same resolution and collision energies.

### Data Analysis

Raw data was analyzed using Skyline (27, 28). MS2 spectra were matched to libraries generated using the synthetic peptides. Extracted ion chromatograms were manually inspected, signal from interfering ions were removed and integration peak boundaries were modified if needed. Precursors with less than 3 valid transitions were excluded from further considerations. To normalize the intensity across runs, two additional peptides from the background matrix INVYYNEATGGK, QSVENDIHGLR from Tubulin beta chain 4b and Type I cytoskeletal keratin 18, respectively, were monitored for the calibration curve. For experiments, precursor intensities were normalized on their respective heavy-labelled peptides.

Further data analysis was performed using in-house R code. Protein-level intensities were obtained by geometrically averaging peptide data.

### Example 2

### Target selection

In order to cover bulk and selective autophagy subtypes, respective literature was screened for relevant proteins (H. Yamamoto, S. Zhang, N. Mizushima, Autophagy genes in biology and disease. Nat Rev Genet 10.1038/s41576-022-00562-w, 1-19 (2023). T. Lamark, T. Johansen, Mechanisms of Selective Autophagy. Annu Rev Cell Dev Biol 37, 143-169 (2021). M. Bordi, R. De Cegli, B. Testa, R. A. Nixon, A. Ballabio, F. Cecconi, A gene toolbox for monitoring autophagy transcription. Cell Death Dis 12, 1044 (2021). D. Siva Sankar, J. Dengjel, Protein complexes and neighborhoods driving autophagy. Autophagy 17, 2689-2705 (2021)). In addition, selected, autophagy-relevant proteins from ongoing research projects were included (J. Zhou, N. L. Rasmussen, H. L. Olsvik, V. Akimov, Z. Hu, G. Evjen, S. Kaeser-Pebernard, D. S. Sankar, C. Roubaty, P. Verlhac, N. van de Beck, F. Reggiori, Y. P. Abudu, B. Blagoev, T. Lamark, T. Johansen, J. Dengjel, TBK1 phosphorylation activates LIR-dependent degradation of the inflammation repressor TNIP1. J Cell Biol 222 (2023)). The chosen targets include proteins relevant to autophagy regulation (ribosomal S6 Kinase and TFEB), the initiation complex (ULK1, RB1CC1/FIP200, ATG13), autophagosome biogenesis (ATG2A, ATG9A, WIPI2, BNIP1, WBP2), the ATG8 lipidation machinery (ATG3, ATG7, ATG12, ATG5, ATG4A) and mammalian ATG8s (GABARAPL1, GABARAPL2), as well as SARs (SQSTM1, NBR1, Optineurin, NDP52, TAX1BP1, Alfy/WDFY3, CCPG1, FAM234B, RTN3, NIX, TOLLIP, FKBP8, BNIP3, AMBRA1, PHB2, NCOA4, STBD1, ATGL) (Table 1).

**Table 2: Target proteins monitored by PRM**

| **Protein Description** | **UniProt Accession** | **Gene name** | **Details** |
|---|---|---|---|
| Autophagy-related protein 2 homolog A | Q2TAZ0 | ATG2A | Lipidation machinery |
| Ubiquitin-like-conjugating enzyme ATG3 | Q9NT62 | ATG3 | Lipidation machinery |
| Ubiquitin-like modifieractivating enzyme ATG7 | O95352 | ATG7 | Lipidation machinery |
| Autophagy-related protein 9A | Q7Z3C6 | ATG9A | Autophagosome biogenesis |
| Vesicle transport protein SEC20 | Q12981 | BNIP1 | Autophagosome biogenesis |
| Gamma-aminobutyric acid receptor-associated protein-like 1 | Q9H0R8 | GABARA PL1 | Mammalian Atg8 protein |
| Gamma-aminobutyric acid receptor-associated protein | 095166 | GABARA P | Mammalian Atg8 protein |
| Microtubule-associated proteins 1A/1B light chain 3A | Q9H492 | MAP1LC3 A | Mammalian Atg8 protein |
| Microtubule-associated proteins 1A/1B light chain 3C | Q9BXW4 | MAP1LC3 C | Mammalian Atg8 protein |
| Transcription factor EB | P19484 | TFEB | Lysosomal biogenesis |
| WD repeat domain phosphoinositideinteracting protein 2 | Q9Y4P8 | WIPI2 | Autophagosome biogenesis |
| Next to BRCA1 gene 1 protein | Q14596 | NBR1 | Ubiquitylated substrate receptor, pexophagy receptor |
| Sequestosome-1 | Q13501 | SQSTM1 | Ubiquitylated substrate receptor |
| Ubiquitin-like protein ATG12 | 094817 | ATG12 | Lipidation machinery |
| Calcium-binding and coiled-coil domain-containing protein 2 | Q13137 | CALCOC O2 | Ubiquitylated substrate receptor |
| Autophagy protein 5 | Q9H1Y0 | ATG5 | Lipidation machinery |
| Cysteine protease ATG4A | Q8WYN0 | ATG4A | Lipidation machinery |
| Ribosomal protein S6 kinase beta-1 | P23443 | RPS6KB1 | Signaling |
| Serine/threonine-protein kinase ULK1 | O75385 | ULK1 | Signaling, initiation |
| RB1-inducible coiled-coil protein 1 | Q8TDY2 | RB1CC1 | Autophagy initiation |
| Cysteine protease ATG4B | Q9Y4P1 | ATG4B | Lipidation machinery |
| Nuclear fragile X mental retardation-interacting protein 1 | Q9UHK0 | NUFIP1 | Proposed ribophagy receptor |
| Starch-binding domain-containing protein 1 | 095210 | STBD1 | Glycophagy receptor |
| BCL2/adenovirus E1B 19 kDa protein-interacting protein 3-like | O60238 | BNIP3L | Mitophagy receptor, pexophagy receptor |
| BCL2/adenovirus E1B 19 kDa protein-interacting protein 3 | Q12983 | BNIP3 | Mitophagy receptor |
| Cell cycle progression protein 1 | Q9ULG6 | CCPG1 | Reticulophagy receptor |
| Nuclear receptor coactivator 4 | Q13772 | NCOA4 | Ferritinophagy receptor |
| Toll-interacting protein | Q9H0E2 | TOLLIP | Ubiquitylated substrate receptor |
| TNFAIP3-interacting protein 1 | Q15025 | TNIP1 | Autophagy receptor |
| Tax1-binding protein 1 | Q86VP1 | TAX1BP1 | Ubiquitylated substrate receptor |
| WW domain-binding protein 2 | Q969T9 | WBP2 | Autophagosome biogenesis |
| WD repeat and FYVE domain-containing protein 3 | Q8IZQ1 | WDFY3 | Ubiquitylated substrate receptor |
| Reticulophagy receptor FAM 134B | Q9H6L5 | FAM 134B | Reticulophagy receptor |
| Autophagy-related protein 13 | 075143 | ATG13 | Initiation |
| Reticulon-3 | 095197 | RTN3 | Reticulophagy receptor |
| Peptidyl-prolyl cis-trans isomerase FKBP8 | Q14318 | FKBP8 | Mitophagy receptor |
| Gamma-aminobutyric acid receptor-associated protein-like 2 | P60520 | GABARA PL2 | Mammalian Atg8 protein |
| Activating molecule in BECN1-regulated autophagy protein 1 | Q9C0C7 | AMBRA1 | Mitophagy receptor |
| Patatin-like phospholipase domain-containing protein 2 | Q96AD5 | PNPLA2 | Lipophagy receptor |
| Prohibitin-2 | Q99623 | PHB2 | Mitophagy receptor |
| Optineurin | Q96CV9 | OPTN | Ubiquitylated substrate receptor |

Peptides of proteins-of-interest for targeted proteomics are commonly generated by proteolytic digestion using the endoprotease trypsin. Not all tryptic peptides from a given source protein are suited for targeted MS as some sequences might not fit the requirements of LC (highly hydrophilic or hydrophobic peptides), chemical stability (e.g. post-lysis methionine oxidation), or distribution of trypsin cleavage sites (lysine or arginine positions in the sequence prevent digestion into suitable peptides yielding either too large or too small peptides). For these reasons, only peptide sequences between 7 and 25 amino-acids in lengths were considered. Due to the sensitivity to oxidations, methioninecontaining sequences were also excluded. Unfortunately, these restrictions led to the exclusion of some important protein candidates. As an example, human ATG8 proteins are very short proteins that only generate a limited number of tryptic peptides. Among this small set, some peptides needed to be removed as their sequences did not match our quality criteria. As a result, only three peptides from GABARAPL1 and GABARAPL2 were further considered and all LC3s (MAP1LC3A, B, B2 C) did not make it to the final list. As a next step, shortlisted peptides were screened on their detection in previous MS experiments by our group as well as in publicly available MS datasets from PeptideAtlas (17). This led to a selection of 114 peptides sequences from 41 source proteins (Table 1). These were synthesized following the AQUA principle using isotopically labelled arginine or lysine variants (18), supporting their use as synthetic spike-ins in order to aid identifications of endogenous peptides. To assess the usability of these peptides, we first determined their linear concentration range, i.e. the range in which changes in quantities generate a proportional change in the recorded MS signal.

### Peptide characterization

As with all analytical techniques, quantitative accuracy can only be achieved within a certain analyte concentration range. Quantitative accuracy of targeted proteomics assays is achieved by determining the linear range of detection, the limit of detection and the limit of quantification of respective analytes, i.e. the analyte concentrations at which the method generates a signal clearly separated from the noise and a reproducible signal, respectively. These values are peptide-specific and depend in part on the way peptides ionize; hence, they have to be determined experimentally for all of the target analytes by measuring calibration curves using concentration gradients.

The calibration curves for the targeted peptides were measured in five replicates across 11 different concentrations from 3-fold serial dilutions (0.15 fmol to 10'000 fmol on column) and five blank samples. Known amounts of heavy-labeled peptides were spiked into the same tryptic whole-cell-digest from an A549 human lung carcinoma cell line. These spiked digests were then separated into five different samples. Peptide desalting and MS sample preparation was performed separately on each of these samples to reproduce the variation introduced by these steps.

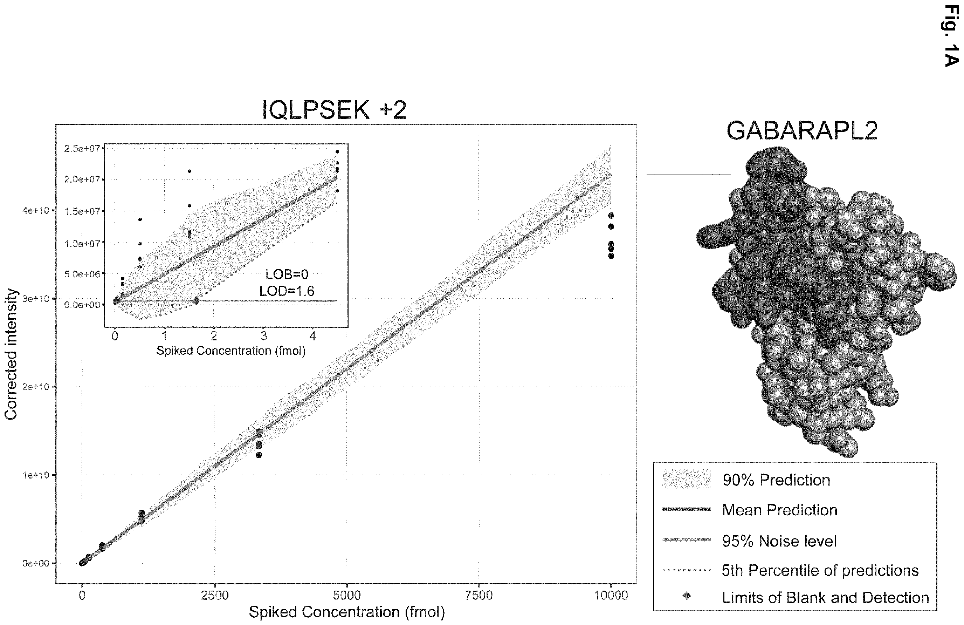

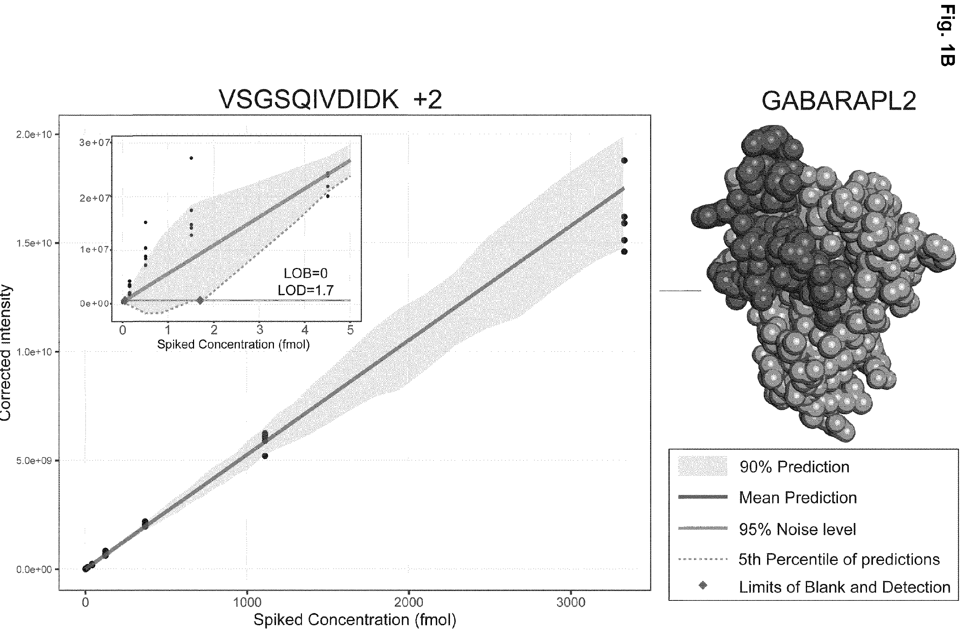

Only peptide precursors for which a minimum of three fragment ions were detected were used for further analyses. As expected, some peptides reached their upper limits of quantification at high concentrations resulting in a loss of linearity of the measured signal or exhibited poor chromatographic results such as tailing. These datapoints were manually removed from further analysis and the last concentration in the linear range was considered the upper limit of quantification for these targets. The remaining points were used to fit a calibration curve using the MSStats LOB/LOD package. Briefly, this method enables to fit a nonlinear regression model to the intensity-concentration response curve. Limits of quantification (LOQ) and detection (LOD) can then be derived from this model. LODs were defined as the concentrations corresponding to the intersection of the upper bound of the intensity prediction interval for the blank samples (corresponding to the noise level) and the mean predicted intensity of spiked amounts. LOQs were set as the concentrations corresponding to the intersection between the upper bound of the predicted noise intensity and the lower bound of the confidence interval of predicted intensities for given concentrations (see Fig. 1 as example). Out of the 114 considered peptides, 17 could either not be reproducibly detected or accurately quantified and were removed from further considerations, leading to a final list of 97 peptides with quantified LODs and LOQs (Table 2).

### Robustness of the assay in a biological context

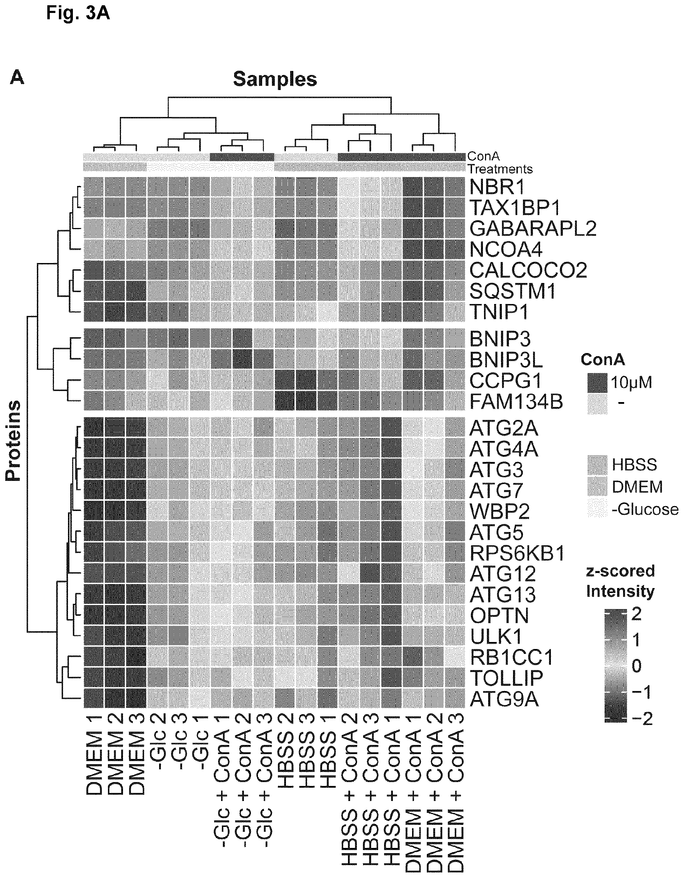

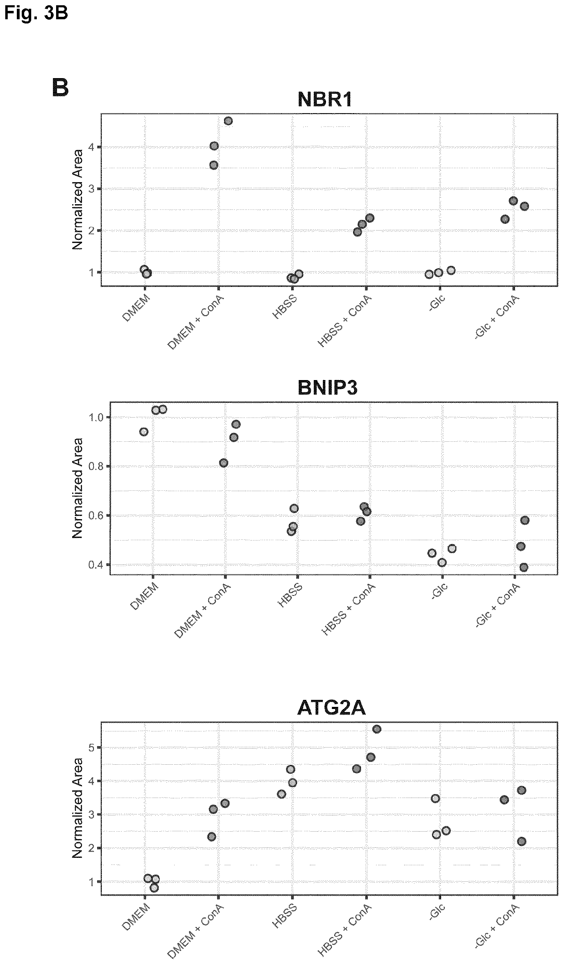

To assess the reproducibility of our approach, a first setup experiment was performed comparing samples under normal growth conditions (DMEM) to samples treated for 2 h by Concanamycin A (DMEM + ConA), which inhibits the V-ATPase and thereby, lysosomal hydrolases (Figure 3). This assay is commonly used to measure autophagy fluxes as proteins which are degraded in lysosomes will accumulate under ConA treatment. The ratio of (DMEM + ConA)/(DMEM) reflects autophagy-dependent protein degradation, i.e. the autophagy flux. Biological triplicates of each condition were prepared and the amount of spike-in peptide was adjusted for each peptide based on the calibration curves, to ensure a stable signal from all standards reflecting respective peptide concentrations.

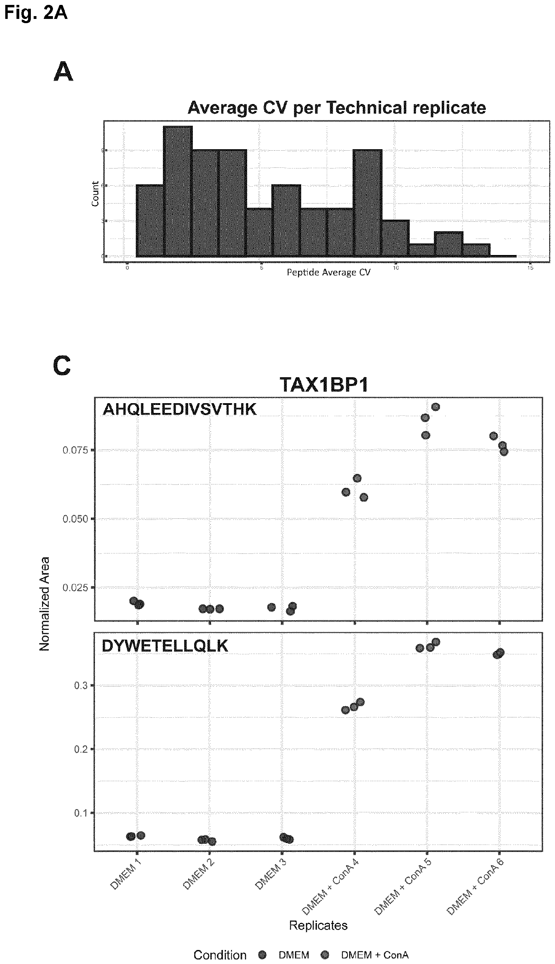

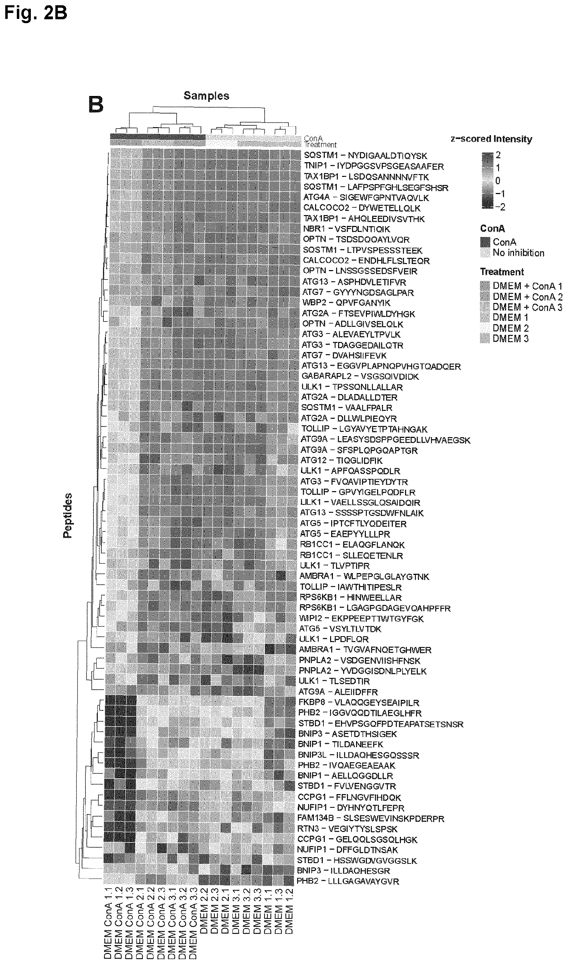

From each biological replicate, three technical replicates were performed to assess technical variation. Peak intensities of light peptides were normalized to their respective heavy-labeled standards. Technical coefficients of variations (CVs) were calculated for each technical triplicate and averaged across all biological replicates. Technical CVs ranged from 0.98% to 12.9% (Figure 4a) depending on the peptides. Following treatment with ConA, the lysosomal hydrolases are inhibited resulting in the accumulation of material which was targeted for recycling through autophagy. This effect could be clearly observed for a variety of well characterized autophagy-related proteins, such as GABARAPL2, SQSTM1, TAX1BP1 and CALCOCO2 but was also noticed for many other ATG proteins for which this effect was not previously described (Fig. 2b, 2c). Thus, proteins involved in autophagy initiation like the ULK1 complex members ULK1, FIP200 and ATG13, as well as proteins involved in ATG8 lipidation like ATG3, ATG4, and ATG5 were also stabilized by ConA, indicating that also autophagy regulators are turned over by lysosomal degradation under growth conditions.

### Assessment of stimulus-dependent protein degradation by autophagy

Having established the technical principles for our assay, it was next determined if it was possible to identify differences in protein regulation of two classical and well described autophagy inducing stimuli, i.e. amino acid and glucose starvation. Both treatments were performed for a duration of 2 h and coupled to the inhibition of lysosomal degradation with ConA to study autophagy flux.

Peptides derived from 38 proteins were detected and accurately quantified. To identify potentially different responses, the results were filtered for proteins showing a significant difference in abundance between the respective treatments (p-value < 0.01 in a one-way ANOVA) yielding a total of 25 differentially regulated proteins. Upon clustering of the results three clear clusters formed (Fig. 3). The top cluster comprised mostly the welldescribed p62-like autophagy receptors, i.e. ubiquitin-dependent soluble SARs, showing a clear flux under all of the treatments, also under basal conditions. This cluster also contained the ATG8-homolog GABARAPL2, a member of the core autophagy machinery functioning as SAR docking site in the autophagosomal membrane. These data imply that under all tested conditions selective targeting of cargo proteins to autophagosomes takes place. As a mammalian ATG8 and defining component of autophagosomes, GABARAPL2 is constitutively turned over, suggesting that receptors following the same pattern are also turned over in a constitutive way. While most of the other proteins assigned to this cluster are known to bind a wide range of ubiquitylated substrates, NCOA4 a selective ferritinophagy receptor was also assigned to this cluster. This could suggest a basal turnover of this receptor probably in an unbound state in conditions in which iron is not limiting, and turnover of the ligand-bound receptor when ferritinophagy is required by the cell.

The second cluster comprises known ubiquitin-independent SARs, such as the mitophagy receptors BNIP3 and NIX, and the reticulophagy receptors CCPG1 and FAM134B. While the overall abundance of these receptors does seem to change upon different treatments, it does not seem to be regulated by autophagy as inhibition of lysosomal degradation does not generate significant changes in their abundances.

The last cluster mostly comprises core proteins involved in autophagy regulation, such as proteins involved in initiation of autophagy, autophagosome biogenesis, and ATG8 lipidation. The proteins assigned to this cluster all show a lower abundance under basal conditions, which seems to be caused by autophagy as this amount increases upon ConA treatment, similarly as observed in the setup experiment (Fig. 2). Upon starvation, ConA treatment ceases to significantly impact the abundance of these proteins. This implies that upon stress, these proteins are spared from degradation to ensure a proper autophagy response.

## Claims

1. A peptide mix comprising peptides for use in monitoring autophagy, the peptides being derived from proteins involved in autophagy.

2. The peptide mix according to claim 1, wherein the peptides are isotopically labelled heavy peptides.

3. The peptide mix according to claim 1 or 2, wherein the proteins are proteins involved in autophagy regulation, in autophagosome initiation, in membrane nucleation, in membrane expansion, in pore closure, in fusion of the autophagosome with lysosomes and/or selective autophagy receptors (SAR) proteins.

4. The peptide mix according to claim 1 to 3, wherein the proteins involved in autophagy regulation are selected from the group consisting of ribosomal S6 kinase (RPS6KB1) and TFEB.

5. The peptide mix according to any of claims 1 to 4, wherein the proteins involved in autophagosome initiation are selected from the group consisting of ULK1, ULK2, RB1CC1/FIP200 and ATG13.

6. The peptide mix according to any of claims 1 to 5, wherein the proteins involved in membrane nucleation are selected from the group consisting of ATG9A, AMBRA1, and WBP2.

7. The peptide mix according to any of claims 1 to 6, wherein the proteins involved in membrane expansion are selected from the group consisting of ATG2A, WIPI1, WIPI2, WIPI3, WIPI4, ATG3, ATG7, ATG12, ATG5 and ATG4A.

8. The peptide mix according to any of claims 1 to 7, wherein the proteins involved in pore closure and fusion with lysosomes are GABARAPL1 or GABARAPL2.

9. The peptide mix according to any of claims 1 to 8, wherein the SAR proteins are selected from the group consisting of SQSTM1, NBR1, Optineurin, CALCOCO2, TAX1BP1, Alfy, CCPG1, FAM134B, RTN3, NIX/BNIP3L, TOLLIP, TNIP1, FKBP8, BNIP3, PHB2, NCOA4, NUFIP1, STBD1, BNIP1, PHBP2 and PNPLA2.

10. The peptide mix according to any of claims 1 to 9, wherein the peptide mix comprises at least 7 peptides selected from the group consisting of peptides having SEQ ID NO: 1 to 97.

11. The peptide mix according to any of claims 1 to 10, wherein the peptide mix comprises at least one peptide derived from a protein involved in autophagy regulation, at least one peptide derived from a protein involved in autophagosome initiation, at least one peptide derived from a protein involved in membrane nucleation, at least one peptide derived from a protein involved in membrane expansion, at least one peptide derived from a protein involved in pore closure, at least one peptide derived from a protein involved in fusion with lysosomes and at least one peptide derived from a selective autophagy receptors (SAR) protein.

12. The peptide mix according to any of claims 1 to 10, wherein the peptide mix consists of at least one peptide derived from each SAR protein selected from the group consisting of SQSTM1, NBR1, Optineurin, CALCOCO2, TAX1BP1, Alfy, CCPG1, FAM134B, RTN3, NIX/BNIP3L, TOLLIP, TNIP1, FKBP8, BNIP3, PHB2, NCOA4, NUFIP1, STBD1, BNIP1, PHBP2 and PNPLA2.

13. Use of the peptide mix according to any of claims 1 to 12 as a peptide standard for mass spectrometry.

14. A method for monitoring autophagy, the method comprising using the peptide mix according to any of claims 1 to 12 as a peptide standard for mass spectrometry.

15. The method according to claim 14, comprising a step of quantifying proteins involved in autophagy by mass spectrometry.

16. The method according to claim 15, wherein the proteins that are quantified are proteins involved in autophagy regulation, in autophagosome initiation, in membrane nucleation, in membrane expansion, in pore closure, in fusion of the autophagosome with lysosomes and/or SAR proteins.
